# EUROPEAN PATENT SPECIFICATION

(11) **EP 1 049 484 B1**
(45) Date of publication and mention of the grant of the patent: **31.03.2004**
(21) Application number: 99902189.2
(22) Date of filing: 12.01.1999
(51) Int. Cl.: A61K 38/20, A61K 48/00, A61P 31/12, A61P 33/00, A61P 35/00

(54) **USE OF IL-11 TO ENHANCE CELL MEDIATED IMMUNITY FOR TREATING VARIOUS VIRAL AND PARASITIC INFECTIONS AND CANCER**
VERWENDUNG VON IL-11 ZUR VERSTÄRKUNG DER ZELLVERMITTELTEN IMMUNITÄT ZUR BEHANDLUNG VERSCHIEDENER VIRALER UND PARASITÄRER INFEKTIONEN UND KREBS
UTILISATION DE L'IL-11 POUR STIMULER L'IMMUNITE INDUITE PAR LES CELLULES POUR TRAITER DES INFECTIONS VIRALES, PARASITAIRES ET LE CANCER

(30) Priority: 23.01.1998 US 12905
(43) Date of publication of application: 08.11.2000
(73) Proprietor: Genetics Institute, LLC, Cambridge, MA 02140 (US)
(72) Inventor: TREPICCHIO, William, L., Andover, MA 01810 (US); BLISS, Judith, L., Portsmouth, New Hampshire 03801 (US); O'TOOLE, Margot, Newton, MA 02160 (US); DORNER, Andrew, J., Lexington, MA 02173 (US)
(74) Representative: Frohwitter, Bernhard, Dipl.-Ing.
(86) International application number: PCT/US1999/000640
(87) International publication number: WO 1999/037322

(56) References cited:
- US-A- 5 648 467
- US-A- 5 700 664
- AIBA Y ET AL: "Development of natural killer cells, B lymphocytes, macrophages, and mast cells from single hematopoietic progenitors in culture of murine fetal liver cells." BLOOD, (1997 NOV 15) 90 (10) 3923-30, XP002102194
- PATENT ABSTRACTS OF JAPAN vol. 017, no. 591 (C-1125), 28 October 1993 & JP 05 178756 A (TORAY IND INC), 20 July 1993

## Description

### FIELD OF INVENTION

The present invention relates generally to the treatment of various viral and parasitic infections and cancer, particularly through enhancing the generation of cytotoxic T cells.

### BACKGROUND OF THE INVENTION

Interleukin-11 [IL-11] is a multifunctional cytokine that has activities on a variety of hematopoietic and immune cell types. In addition to its effects on megakaryocyte, myeloid and erythrocyte progenitors, IL-11 also potently downregulates proinflammatory cytokine production from activated macrophages. Consequently, IL-11 has been shown to reduce inflammation and promote healing in a variety of animal models of acute and chronic inflammation.

Cytotoxic T cells [CTL] are CD8+ effector cells whose primary purpose is to recognize and kill target cells in an MHC class I restricted manner. The mechanism of cytotoxicity is the release of electron-dense cytoplasmic granules as well as the interaction of CD95L (FasL) with CD95 (Fas) on target cells. The immune surveillance properties of CTLs result in host protection from a wide variety of intracellular pathogens such as viruses and parasites. In addition, recognition of tumor antigens by CD8+ T cells can result in the induction of a cell-specific immune response against tumors (Zinkernagel, R.M. And Hengartner, H., Antiviral Immunity Immunology Today 18:258-260 (1997)).

These disorders and their symptoms are briefly summarized below. According to the present invention, IL-11 is to be administered *in vivo* or *ex vivo* to enhance the host CTL response to increase immune surveillance thereby treating the following disorders.

### BRIEF SUMMARY OF THE INVENTION

Surprisingly, the present inventors have found that IL-11 demonstrates the ability to augment the generation of cytotoxic T cells [CTL]. Cells restimulated in the presence of IL-2 and IL-11 demonstrate a significant increase in antigen-specific cytolytic activity over cells restimulated with IL-2 alone. Furthermore, IL-11 could substitute for IL-2 in the generation of CTL activity. The addition of IL-11 during *in vitro* restimulation also resulted in an increased level of Interferonγ [IFNγ] secretion and number of cells producing IFNγ in response to antigen stimulation. In other systems, IL-11 synergized with other factors, such as IL-12, to enhance the number and lytic activity of CTLs. These studies indicate that IL-11 can help in the generation of CTLs and may play a role in the defense against intracellular pathogens. Thus, IL-11 can thus function as a helper factor in the induction of CTL.

The present invention concerns the treatment of a variety of diseases where enhancement of cytotoxic T cell activity has shown to be beneficial including acute viral disorders such as influenza, chronic viral disorders such as Epstein-Barr Virus [EBV], human immunodeficiency virus [HIV], herpes, hepatitis, and varicella zoster, parasitic infections such as malaria and tuberculosis, and cancer. In addition, the present invention discloses the *ex vivo* enhancement of T cell activity from cells derived from patients with the above disorders followed by subsequent reintroduction of the modified cells into the patients.

According to the present invention, IL-11, analogs, and derivatives thereof, are to be administered to patients, either prophylactically or at the onset of symptoms associated with the aforementioned disorders. IL-11 can be administered in suitable pharmaceutically acceptable carriers either alone or in combination with other conventional agents useful in alleviating the symptoms associated with the aforementioned disorders.

In a preferred embodiment, IL-11 can be co-administered with a vaccine against viral or bacterial pathogens, such as an influenza vaccine. In another preferred embodiment, IL-11 can be co-administered with an anti-tumor agent, and can be administered to patients suffereing from cancer or other tumorigenicity.

### DETAILED DESCRIPTION OF THE INVENTION

The present invention concerns the treatment of a variety of diseases where enhancement of cytotoxic T cell activity has shown to be beneficial, including acute viral disorders such as influenza, chronic viral disorders such as EBV, HIB, herpes, hepatitis, and varicella zoster, intracellular pathogens such as malaria and tuberculosis, and cancer. In addition, the present invention discloses the ex vivo enhancement of T cell activity from cells derived from patients with the above diseases followed by subsequent reintroduction of the modified cells into the patients.

Resting B cells, macrophages, CD4+ T cells and CD8+ T cells express the IL-11 receptor alpha-chain, indicating a potential interaction of IL-11 with these cell populations. To understand the role of IL-11 as an immune modulatory protein, its ability to directly interact with various lymphocyte populations was examined. IL-11 directly interacts with an antigen presenting cell [APC], the macrophage, to inhibit LPS-induced proinflammatory cytokine production such as TNF-a, IL-1b and IL-12. IL-11 also interacts directly with purified populations of activated murine CD4+ T cells to enhance or inhibit production of cytokines such as IL-4, IL-5, IL-10, IL-13 and IFN-g. These results indicate an ability of IL-11 to enhance T cell helper activity either directly or indirectly through the APC. Given the ability of IL-11 to modulate effector activity of macrophages and CD4+ T cells, its ability to affect CD8+ cytotoxic T cell effector function either directly or indirectly through the APC or T helper cells was examined. Spleen cells from influenza virus immunized Balb/c mice were isolated and restimulated *in vitro* for seven days with influenza derived MHC class I restricted peptide. Cells restimulated in the presence of both IL-2 and rhIL-11 demonstrated a significant increase in antigen-specific cytolytic activity over cells restimulated in the presence of IL-2 alone. Also, cells restimulated in the presence of rhIL-11 alone demonstrated a significant increase in antigen-specific cytolytic activity. This indicates that rhIL-11 can substitute for the requirement of exogenous IL-2 in this system. The addition of rhIL-11 during *in vitro* restimulation also resulted in an increased level of both antigen driven IFNγ production and number of IFNγ secreting cells.

Interleukin-11 [IL-11] is apleiotropic cytokine that stimulates primitive lymphohematopoietic progenitor cells and synergizes with other hematopoietic growth factors to stimulate the proliferation and maturation of megakaryocytes. IL-1 is described in detail WO91/07495; as well as in U.S. Patent No. 5,215,895; issued June 1, 1993. A cloned human IL-11 was previously deposited with the ATCC, 12301 Parklawn Drive, Rockville, Maryland, on March 30, 1990 under ATCC No. 68284. Moreover, as described in U.S. Patent No. 5,270,181; issued December 14, 1993; and U.S. Patent No. 5,292,646; issued March 8, 1994; IL-11 may also be produced recombinantly as a fusion protein with another protein. IL-11 can be produced in a variety of host cells by resort to now conventional genetic engineering techniques. In addition, IL-11 can be obtained from various cell lines, for example, the human lung fibroblast cell line, MRC-5 (ATCC Accession No. CCL 171) and Paul *et al*., the human trophoblastic cell line, TPA30-1 (ATCC Accession No. CRL 1583). Described in Proc Natl Acad Sci USA *87*:7512 (1990) is a cDNA encoding human IL-I 1 as well as the deduced amino acid sequence (amino acids 1 to 199). U.S. Patent No. 5,292,646, *supra,* describes a des-Pro form of IL-11 in which the N-terminal proline of the mature form of IL-11 (amino acids 22-199) has been removed (amino acids 23-199). As is appreciated by one skilled in the art, any form of IL-11, which retains IL-11 activity, is useful according to the present invention.

In addition to recombinant techniques, IL-11 may also be produced by known conventional chemical synthesis. Methods for constructing the polypeptides useful in the present invention by synthetic means are known to those of skill in the art. The synthetically constructed cytokine polypeptide sequences, by virtue of sharing primary, secondary, or tertiary structural and conformational characteristics with the natural cytokine polypeptides are anticipated to possess biological activities in common therewith. Such synthetically constructed cytokine polypeptide sequences or fragments thereof, which duplicate or partially duplicate the functionality thereof may also be used in the method of this invention. Thus, they may be employed as biologically active or immunological substitutes for the natural, purified cytokines useful in the present invention.

Modifications in the protein, peptide or DNA sequences of these cytokines or active fragments thereof may also produce proteins which may be employed in the methods of this invention. Such modified cytokines can be made by one skilled in the art using known techniques. Modifications of interest in the cytokine sequences, *e*.*g*., the IL-11 sequence, may include the replacement, insertion or deletion of one or more selected amino acid residues in the coding sequences. Mutagenic techniques for such replacement, insertion or deletion are well known to one skilled in the art. (See, *e*.*g*., U. S. Patent No. 4,518,584.)

Other specific mutations of the sequences of the cytokine polypeptides which may be useful therapeutically as described herein may involve, *e.g*., the insertion of one or more glycosylation sites. An asparagine-linked glycosylation recognition site can be inserted into the sequence by the deletion, substitution or addition of amino acids into the peptide sequence or nucleotides into the DNA sequence. Such changes may be made at any site of the molecule that is modified by addition of O-linked carbohydrate. Expression of such altered nucleotide or peptide sequences produces variants which may be glycosylated at those sites.

Additional analogs and derivatives of the sequence of the selected cytokine which would be expected to retain or prolong its activity in whole or in part, and which are expected to be useful in the present method, may also be easily made by one of skill in the art. One such modification may be the attachment of polyethylene glycol (PEG) onto existing lysine residues in the cytokine sequence or the insertion of one or more lysine residues or other amino acid residues that can react with PEG or PEG derivatives into the sequence by conventional techniques to enable the attachment of PEG moieties.

Additional analogs of these selected cytokines may also be characterized by allelic variations in the DNA sequences encoding them, or induced variations in the DNA sequences encoding them. It is anticipated that all analogs disclosed in the above-referenced publications, including those characterized by DNA sequences capable of hybridizing to the disclosed cytokine sequences under stringent hybridization conditions or non-stringent conditions (Sambrook *et al*., Molecular Cloning. A Laboratory Manual, 2d edit., Cold Spring Harbor Laboratory, New York (1989)) will be similarly useful in this invention.

In addition, it is within the art to generate peptide ligands or small molecule analogs of IL-11 which are able to interact with the IL-1 receptor, and thus block the effects [antagonists]or produce the same or similar effects [agonists] as those of IL-11. Methods for generating and assaying such ligands and small molecules are disclosed in PCT Patent Publication WO96/19574. Such ligands and small molecule analogs are also useful in the present invention.

Also considered useful in these methods are fusion molecules, prepared by fusing the sequence or a biologically active fragment of the sequence of one cytokine to another cytokine or proteinaceous therapeutic agent, *e*.*g*., IL-11 fused to IL-6 (see, *e.g.,* methods for fusion described in PCT/US91/06186 (WO92/04455), published March 19, 1992). Alternatively, combinations of the cytokines may be administered together according to the method.

The present invention thus involves treating patients having acute viral disorders such as influenza, chronic viral disorders such as EBV, HIV, herpes, hepatitis, and varicella zoster, parasitic infections such as malaria and tuberculosis and cancer and involves administering an effective amount of IL-11 in a pharmaceutical carrier. Treatment may be therapeutic or prophylactic, or may be at the onset of symptoms associated with the aforementioned disorders.

In a preferred embodiment, IL-11 is to be co-administered with a vaccine against viral or bacterial pathogens, such as those which cause the above disorders. In another preferred embodiment, IL-11 is to be co-administered with an anti-tumor agent, and can be administered to patients suffereing from cancer or other tumorigenicity.

Suitable pharmaceutically acceptable carriers facilitate administration of IL-11 and are well known in the art. Exemplary carriers include sterile saline, lactose, sucrose, calcium phosphate, gelatin, dextrin, agar, pectin, peanut oil, olive oil, sesame oil, and water. Additionally, the carrier or diluent includes a time delay material, such as glyceryl monostearate or glyceryl distearate alone or with a wax. In addition, slow release polymer formulations can be used. Suitable sustain-release matrices contain the active ingredient in a mixture with one or more of the following: sodium bentonite, ethylcellulose, stearic acid, calcium stearate, adipic acid, fumaric acid, polyethylene glycol, deacetylated chitin, and cellulose acetate. Suitable preservatives and/or stabilizers may be included.

Alternatively, IL-11 can be combined with other biological factors and/or conventional agents useful in alleviating the symptoms associated with the aforementioned disorders, as is readily apparent to one skilled in the art. For example, IL-11 may be coadministered with CSFs, such as GCSF, GMCSF or MCSF, with erythropoietin, hepatopoietin, or Interleukins, such as IL-1, IL-2, IL-3, IL-4, IL-5, IL-6, IL-7, IL-8, IL-9, IL-10, IL-12, IL-13, IL-14 or IL-15, or with receptors to any of the above molecules. Preferred embodiments involve coadministration with IL-12, IL-6 or IL-2. A suitable IL-11 formulation comprises, for example, 5 mg IL-11, 3.10 mg histidine, and 22.5 mg glycine, for example, as a lyophilized powder which can be reconstituted with I ml sterile water for injection. As is apparent to one skilled in the art, other suitable formulations are equally effective according to the methods of the present invention.

In the treatment of the aforementioned disorders, IL-11 can be administered by any suitable route, but certain routes are preferable for certain disorders, *e.g.*, administered systemically, *i*.*e*., parenterally. Of the parenteral routes, subcutaneous and intravenous are preferred. IL-11 can be supplied intravenously or can be applied topically in formulations to the nasal mucosa or the conjunctiva or oral mucosa as an aqueous drop formulation or mouthwash, respectively. In localized surface reactions, a topical formulation is preferred whereas in more severe generalized body-wide disorders, parental routes, such as subcutaneous or intravenous injection are preferred. Suitable doses of IL-11 are in the range of 1-50µg/kg subcutaneous for multiple daily doses and for shorter periods of treatment in severe T-cell states, doses are increased to 50 to 100µg/kg subcutaneous or intravenous. Generally, a suitable dose of cytokine, *e.g.*, IL-11 ranges broadly, preferably between 1 and 100µg/kg body weight. Another suitable dose may be in the range of about 10 to 50µg/kg and about 50µg/kg with a preferable amount of 25µg IL-11 per kilogram of body weight. If desired, these doses can be adjusted to units. A unit is conventionally described as the concentration of polypeptide which leads to half-maximal stimulation in a suitable assay, *e.g.*, for IL-11, the T1165 assay described in WO 91/07495. Doses may be administered daily for between one day and six months, or for as long as is deemed necessary and safe, as is readily ascertained by standard tests by the attending physician, depending upon the nature of the disorder being treated. Where appropriate, the dosages may be adjusted upward or downward, for example, a dosing regimen requiring administration of IL-11 at a dose of 25 µg/kg, daily for one week, or fewer days, or multiple weeks if indicated. The progress of treatment is appropriately monitored by measurement of markers associated with the disorder being treated to determine if such a dose results in an increase of for example, perform (or corresponding marker) and if not, increasing the dose two-fold for an additional time period of treatment and measurement of marker levels until an effective dosing regimen is reached.

An alternative mode of therapeutic treatment is the *ex vivo* expansion of CTLs from affected patients. For example, peripheral blood mononuclear cells [PBMC] can be isolated from the blood of patients and stimulated in vitro with different doses of rhIL-11 to enhance the generation of CTLs. Appropriate in vitro doses of 10 ng/ml -500 ng/ml are desirable. Cytotoxic T cell assays can be performed to determine the maximal efficacious dose. Cells can then be subsequently reinfused into the patient.

The following examples illustrate the present invention and in particular the use of IL-11 for the manufacture of a medicament for treating acute viral infections such as influenza, chronic viral infections such as EBV, HIV, herpes, hepatitis, and varicella zoster, parasitic infections such as malaria and tuberculosis and cancer. In addition, provided by the present invention are the ex vivo enhancement of T cell activity from cells derived from patients with the above T cell disorders followed by subsequent reintroduction of the modified cells into the patients. However, many modifications and alternatives of the invention will be apparent to the skilled in the art having read the present specification. For example, it will be understood by those of skill in the art that IL-11 encompasses the protein produced by the sequences presently disclosed in the art, as well as proteins characterized by the modifications described above which retain substantially similar activity to IL-11. In addition, it will be recognized that the modifications, analogs of IL-11 described above are useful in the methods of the present invention. Thus, the examples do not limit the scope of the invention in any way.

### Example 1- The IL-11 receptor alpha chain is expressed on B and T lymphocytes

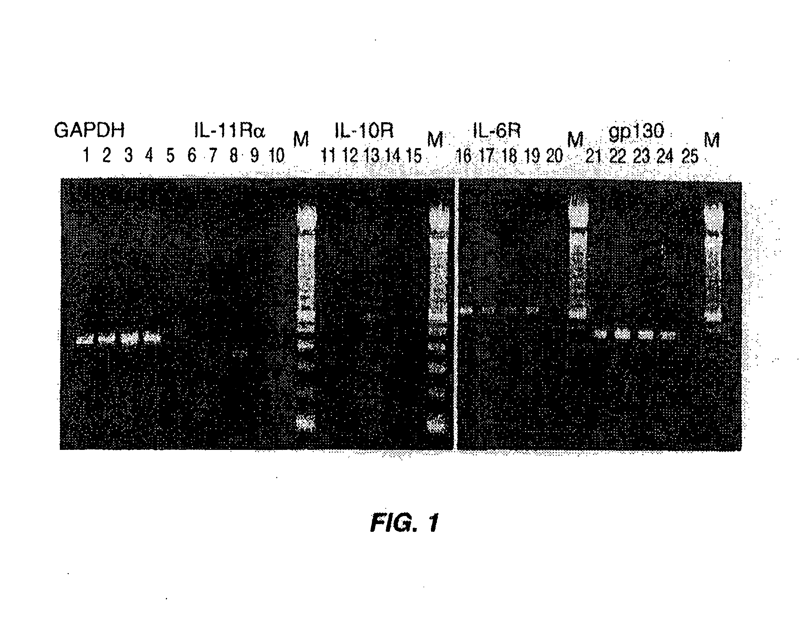

Murine B Cells and CD4+ and CD8+ T cells were purified from Balb/c spleens using positive selection with MACS (Magnetic Cell Sorting) Microbeads conjugated to anti-mouse B220, CD4 or CD8 antibodies, as per manufacturers protocol (Miltenyi Biotec, Sunnyvale, CA). RNA was extracted from purified cell populations described above using RNA Stat-60 (Tel-test, Inc., TX) as per manufacturer's protocol. RNA was DNase treated (RQ1 DNase, Promega, Madison, WI) for 30 min at 37°C, then heat inactivated for 5 min at 75°C. RT-PCR was performed (GeneAmp RNA PCR Kit, Perkin Elmer) using 40ng RNA (10ng for GAPDH) and oligo pairs specific for murine GAPDH, IL-11 receptor a chain, IL-10 receptor, IL-6 receptor or gp130, and visualized on an ethidium-stained agarose gel (shown). PCR reactions on RNA samples in the absence of reverse transcriptase were negative for each oligo pair, and served as a control for DNA contamination. The example indicates that the IL-11 receptor alpha chain mRNA is detected in highly purified populations of CD4+, CD8+ and B220+ lymphocytes. [See Figure 1]

### Example 2 - IL-11 Increases Antigen Specific Cytolytic Activity in Bulk Spleen Cultures.

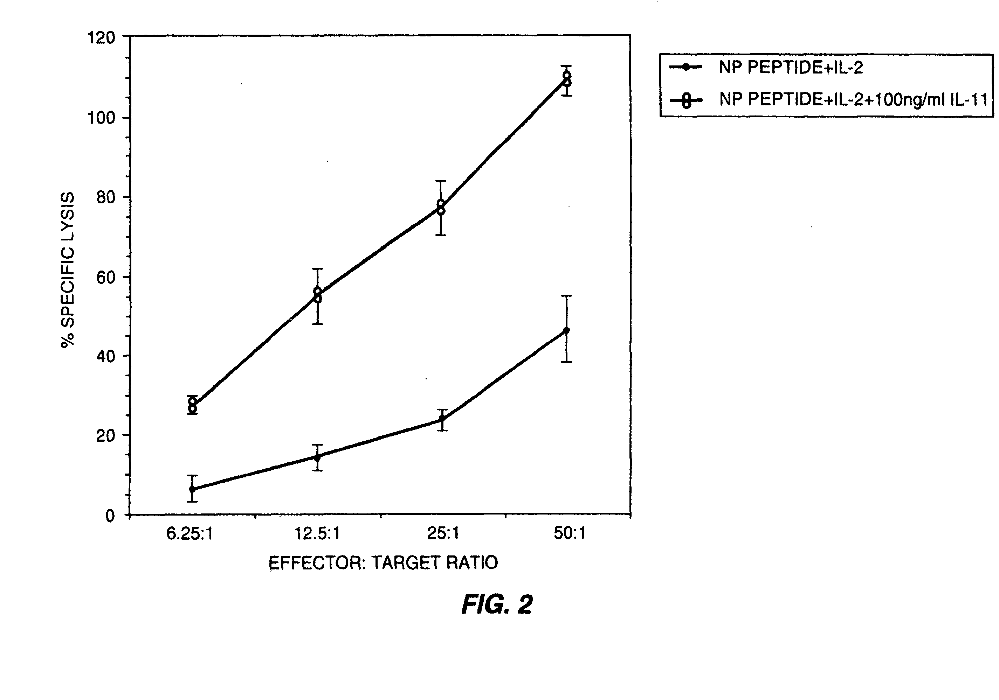

Seven day splenic bulk cultures from cells treated with IL-2, or IL-2 and IL-11,were serially diluted and added to 1x10^{4 51}Cr-labelled P815.P1HTR cells, which were pulsed for one hour in the presence or absence of NP peptide. The cells were incubated for 4 hours. Supernatants were harvested, and ⁵¹Cr release was determined. Maximum lysis on the target cells was induced with 2% Triton-X 100. % lysis = [(CPM-spontaneous CPM of NP peptide pulsed targets)/(maximum CPM of NP peptide pulsed targets- spontaneous CPM of NP peptide pulsed targets)]X100. % specific lysis=(% lysis on NP peptide pulsed target cells)-(% lysis of control target cells). The example indicates that IL-11 can significantly enhance the % specific lytic activity of NP peptide-specific cytotoxic T cells. At a 50:1 effector to target cell ratio, a 250% enhancement in % specific lysis is observed in cells treated with IL-2 and IL-11 versus cells treated with IL-2 alone. [See Figure 2]

### Example 3- IL-11 can Substitute for the Requirement of Exogenous IL-2 in the Generation of Antigen Specific Cytolytic Activity in Bulk Spleen Cultures.

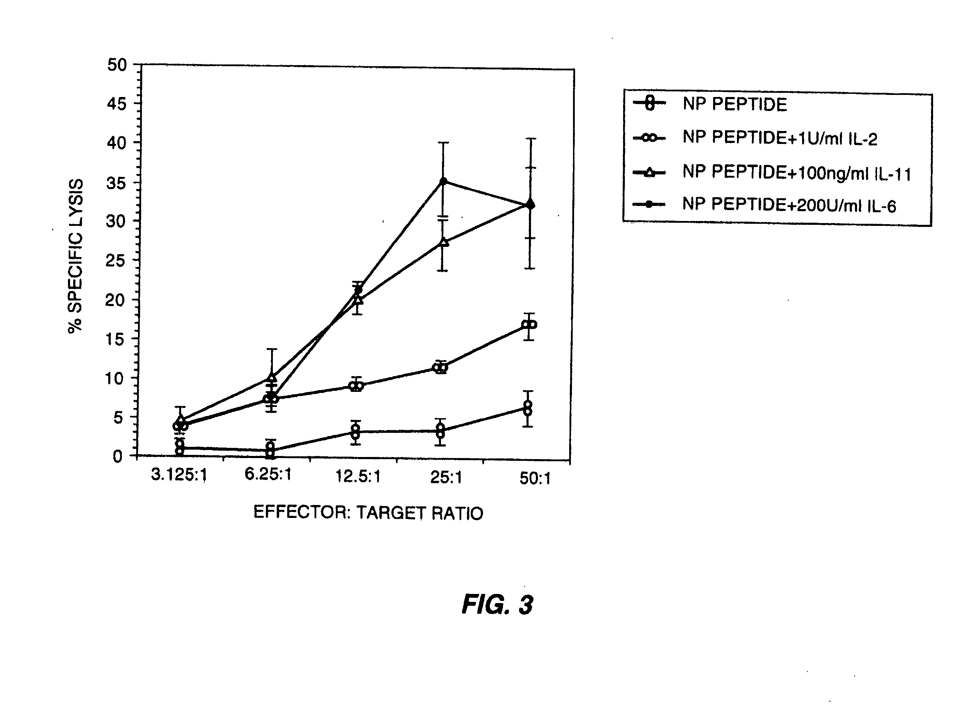

Seven day splenic bulk cultures from cells treated with muIL-2, muIL-6, or rhIL-11 were serially diluted and added to 1x10^{4 51}Cr-labelled P815.P1HTR cells, which were pulsed for one hour in the presence or absence of NP peptide. The cells were incubated for 4 hours. Supernatants were harvested, and ⁵¹Cr release was determined. % specific lysis was calculated as described in Figure 3. The example indicates that IL-11 treatment of cells alone in the absence of exogenous IL-2 can significantly enhance the % lytic activity of NP peptide-specific cytotoxic T cells. At a 50:1 effector to target ratio, a 7 fold increase in % specific lysis is observed between cells treated with IL-11 versus cells treated with no exogenously added cytokines. [See Figure 3]

### Example 4 - IL-11 Increases the Number of Influenza Specific IFNγ- Producing Cells.

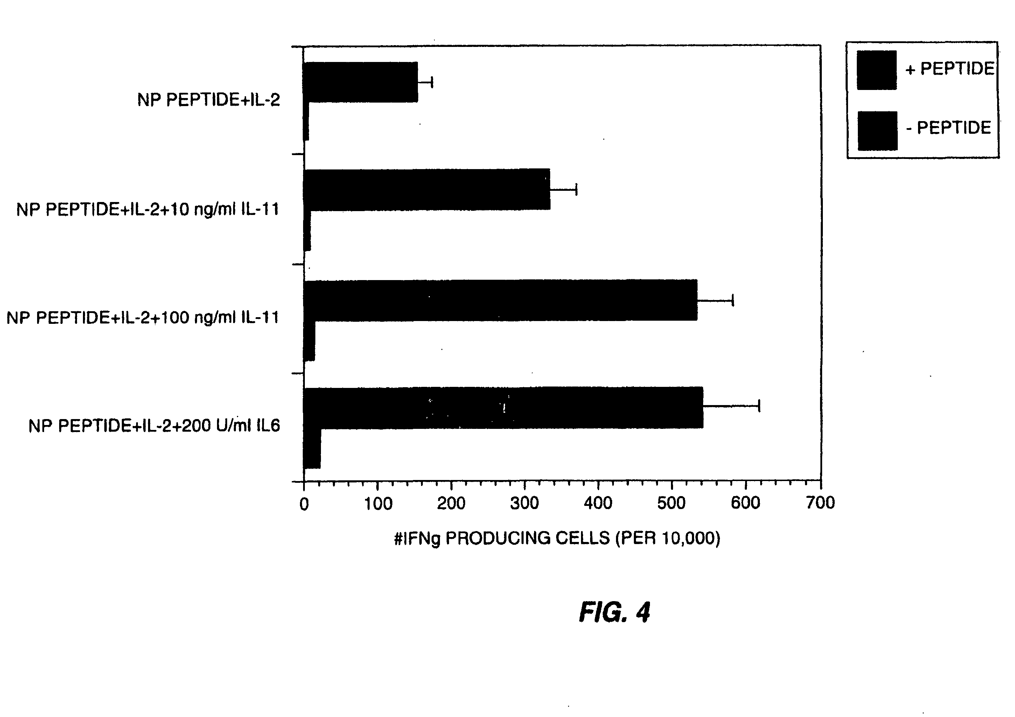

Sterile flat bottom 96 well plates (Costar) were coated with an anti-IFNγ antibody (R46A2, 10 µg/ml, 50 ul/well) at 37°C for 2 hrs and blocked with PBS+1%BSA+0.05% Tween 20 at 37°C for 1 hour and washed with PBS. Serial dilutions of splenic cells diluted in RPMI+10% FCS were added to the plates and incubated for 16 hr at 37°C, and then washed 6 times. Biotinylated anti-IFNγ antibody XGM.1 (1.19 µg/ml) was added to each well and incubated 90 min at room temperature. Following another 4 washes, streptavidin alkaline phosphatase was added and incubated 1 hr at room temperature. After 4 washes, the plates were developed with a solution of 0.6% low melting agarose in 0.1M 2-amino-2-methyl-1 propanol, pH10.5, containing 1mg/ml BCIP (5-Bromo-4-Chloro-3-Indonyl phosphate). The plates were incubated for 16 hr at room temperature and then the spots were counted. The example indicates that IL-11 enhances the number of influenza -specific IFN-g producing cytotoxic T cells. Cells treated with either IL-2 and either 10 ng/ml or 100 ng/ml of IL-11 had 2-3.5 fold increased number of IFN-g producing cells compared to cells treated with IL-2 alone. [See Figure 4]

### Example 5 - IL-11 Increases IFNγ Production from Influenza Specific CTLs.

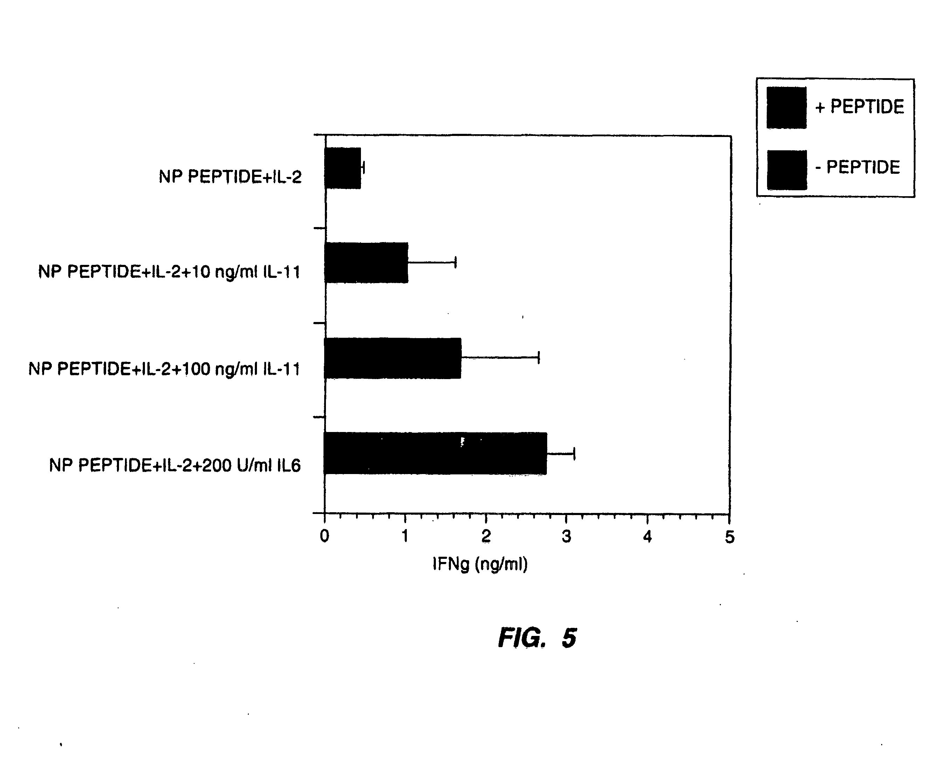

ELISA plates (EIA capture plates, Costar) were coated with an anti-IFNγ antibody (R46A2. 3 µg/ml, 50 ul/well) at 4°C for 16 hr and blocked with THSG (Tris high salt gelatin) at 37°C for 2 hours and washed 4 times. Serial dilutions of IFNγ control supernatants and of unknown samples diluted in PBS+10% FCS were added to the plates and incubated for 2 hr at room temperature, and then washed 4 times. Biotinylated antibody XGM.1 (1.19 µg/ml) was added to each well and incubated 90 min at room temperature. Following another 4 washes, peroxide-conjugated Avidin (Vector) was added and incubated 1 hr at room temperature. After 4 washes, the plates were developed with ABTS (Kirkegaard and Perry) for 9 min in the dark and stopped with 1 % SDS. The absorbance at 405 nm was determined with a Vmax kinetic microplate reader (Molecular Devices). The example indicates that IL-11 can enhance the amount of IFN-g produced from influenza-specific cytotoxic T cells. 2-4 fold elevated levels of IFN-g were detected in the conditioned medium of cells treated with IL-2 and either 10 ng/ml or 100 ng/ml IL-11. [See Figure 5]

While the present invention has been described in terms of specific methods and compositions, it is understood that variations and modifications will occur to those skilled in the art upon consideration of the present invention. Numerous modifications and variations in the invention as described in the above illustrative examples are expected to occur to those skilled in the art and, consequently, only such limitations as appear in the appended claims should be placed thereon.

## Claims

1. An *in vitro* method of inducing cytotoxic T cell production, wherein cytotoxic T cells undergo at least one of:
(a) treatment with IL-11 protein;
(b) transfection with a DNA molecule encoding an IL-11 protein, and the cells are cultured under conditions wherein IL-11 protein is produced.

2. An *in vitro* method of inducing enhancement of cytotoxic T cell activity, comprising administering IL-11 to a suitable T cell population.

3. Use of IL-11 for the preparation of a medicament for inducing cytotoxic T cell production or inducing enhancement of cytotoxic T cell activity in a patient who suffers from at least one of a chronic viral disorder selected from the group comprising Epstein-Barr Virus [EBV], human immunodeficiency virus [HIV], herpes, hepatitis, varicella zoster or a parasitic infection selected from the group comprising malaria and tuberculosis

4. The use of IL-11 for the manufacture of a medicament for inducing cytotoxic T cell production in a patient wherein cytotoxic T cells are treated with IL-11 protein *ex vivo*, and wherein said patient suffers from at least one of:
(a) an acute viral disorder;
(b) influenza;
(c) a chronic viral disorder;
(d) a chronic viral disorder selected from the group comprising Epstein-Barr Virus [EBV], human immunodeficiency virus [HIV], herpes, hepatitis and varicella zoster;
(e) a parasitic infection;
(f) a parasitic infection selected from the group comprising malaria and tuberculosis; and
(g) cancer.

5. The use of IL-11 for the manufacture of a medicament for inducing enhancement of cytotoxic T cell activity in a patient comprising administering IL-11 to an *ex vivo* cytotoxic T cell population of said patient, and wherein said patient suffers from at least one of:
(a) an acute viral disorder:
(b) influenza:
(c) a chronic viral disorder:
(d) a chronic viral disorder selected from the group comprising Epstein-Barr Virus [EBV]. human immunodeficiency virus [HIV], herpes, hepatitis and varicella zoster.
(e) a parasitic infection:
(f) a parasitic infection selected from the group comprising malaria and tuberculosis; and
(g) cancer.

6. The use of a DNA molecule encoding an IL-11 protein for the manufacture of a medicament for inducing cytotoxic T cell production in a patient, wherein a cytotoxic T cell is transfected with said DNA molecule and said cell is cultured *ex vivo* under conditions wherein IL-11 protein is produced, and wherein said patient suffers from at least one of:
(a) an acute viral disorder;
(b) influenza;
(c) a chronic viral disorder;
(d) a chronic viral disorder selected from the group comprising Epstein-Barr Virus [EBV], human immunodeficiency virus [HIV], herpes, hepatitis and varicella zoster; and
(e) a parasitic infection;
(f) a parasitic infection selected from the group comprising malaria and tuberculosis; and
(g) cancer.

## Patentansprüche

1. In vitro Verfahren zur Induktion der Produktion zytotoxischer T-Zellen, wobei zytotoxische T-Zellen
(a) mit IL-11 Protein behandelt werden und/oder
(b) mit einem für ein IL-11 Protein kodierenden DNA-Molekül transfiziert werden, und die Zellen unter Bedingungen, bei denen IL-11 Protein produziert wird, kultiviert werden.

2. In vitro Verfahren zur Induktion der Verstärkung der Aktivität von zytotoxischen T-Zellen, umfassend das Verabreichen von IL-11 einer geeigneten T-Zell-Population.

3. Verwendung von IL-11 zur Herstellung eines Arzneimittels zur Induktion der Produktion zytotoxischer T-Zellen oder der Induktion der Verstärkung der Aktivität von zytotoxischen T-Zellen in einem Patienten, welcher an mindestens einer chronischen viralen Erkrankung, ausgewählt aus der Gruppe, umfassend Epstein-Barr-Virus [EBV], Humanimmundefizienzvirus [HIV], Herpes, Hepatitis, Varizella zoster, oder einer parasitären Infektion, ausgewählt aus der Gruppe, umfassend Malaria und Tuberkulose, leidet.

4. Verwendung von IL-11 zur Herstellung eines Arzneimittels zur Induktion der Produktion zytotoxischer T-Zellen in einem Patienten, wobei zytotoxische T-Zellen ex vivo mit IL-11 Protein behandelt werden, und wobei der Patient an mindestens einer Erkrankung leidet aus:
(a) einer akuten viralen Erkrankung,
(b) Grippe,
(c) einer chronischen viralen Erkrankung,
(d) einer chronischen viralen Erkrankung, ausgewählt aus der Gruppe, umfassend Epstein-Barr-Virus [EBV], Humanimmundefizienzvirus [HIV], Herpes, Hepatitis und Varizella zoster,
(e) einer parasitären Infektion,
(f) einer parasitären Infektion, ausgewählt aus der Gruppe, umfassend Malaria und Tuberkulose, und
(g) Krebs.

5. Verwendung von IL-11 zur Herstellung eines Arzneimittels zur Induktion der Verstärkung der Aktivität zytotoxischer T-Zellen in einem Patienten, umfassend das Verabreichen von IL-11 einer ex vivo Population zytotoxischer T-Zellen des Patienten, und wobei der Patient an mindestens einer Erkrankung leidet aus:
(a) einer akuten viralen Erkrankung,
(b) Grippe,
(c) einer chronischen viralen Erkrankung,
(d) einer chronischen viralen Erkrankung, ausgewählt aus der Gruppe, umfassend Epstein-Barr-Virus [EBV], Humanimmundefizienzvirus [HIV], Herpes, Hepatitis und Varizella zoster,
(e) einer parasitären Infektion,
(f) einer parasitären Infektion, ausgewählt aus der Gruppe, umfassend Malaria und Tuberkulose, und
(g) Krebs.

6. Verwendung eines für ein IL-11 Protein kodierenden DNA-Moleküls zur Herstellung eines Arzneimittels zur Induktion der Produktion zytotoxischer T-Zellen in einem Patienten, wobei eine zytotoxische T-Zelle mit dem DNA-Molekül transfiziert wird und die Zelle ex vivo unter Bedingungen kultiviert wird, bei denen IL-11 Protein produziert wird, und wobei der Patient an mindestens einer Erkrankung leidet aus:
(a) einer akuten viralen Erkrankung,
(b) Grippe,
(c) einer chronischen viralen Erkrankung,
(d) einer chronischen viralen Erkrankung, ausgewählt aus der Gruppe, umfassend Epstein-Barr-Virus [EBV], Humanimmundefizienzvirus [HIV], Herpes, Hepatitis und Varizella zoster, und
(e) einer parasitären Infektion,
(f) einer parasitären Infektion, ausgewählt aus der Gruppe, umfassend Malaria und Tuberkulose, und
(g) Krebs.

## Revendications

1. Procédé *in vitro* pour induire la production de cellules T cytotoxiques, dans lequel les cellules T cytotoxiques subissent au moins une des opérations suivantes :
(a) un traitement à l'aide de la protéine IL-11 ;
(b) une transfection à l'aide d'une molécule d'ADN codant une protéine IL-11, les cellules étant cultivées dans des conditions dans lesquelles la protéine IL-11 est produite.

2. Procédé *in vitro* pour induire une augmentation de l'activité des cellules T cytotoxiques, comprenant le fait d'administrer IL-11 à une population de cellules T appropriée.

3. Utilisation de IL-11 pour la préparation d'un médicament destiné à induire la production de cellules T cytotoxiques ou à induire une augmentation de l'activité des cellules T cytotoxiques, chez un patient qui souffre d'au moins une des deux affections suivantes : un trouble chronique d'origine virale choisi dans l'ensemble constitué par le virus d'Epstein-Barr [VEB], le virus de l'immunodéficience humaine [VIH], l'herpès, l'hépatite, la varicelle-zoster ou une infection d'origine parasitaire choisie dans l'ensemble constitué par la malaria et la tuberculose.

4. Utilisation de IL-11 pour la fabrication d'un médicament destiné à induire la production de cellules T cytotoxiques chez un patient dont on a traité, *ex vivo,* les cellules T cytotoxiques à l'aide de la protéine IL-11, ledit patient souffrant d'au moins une des affections suivantes :
(a) un trouble viral aigu ;
(b) la grippe ;
(c) un trouble viral chronique ;
(d) un trouble viral chronique choisi dans l'ensemble constitué par le virus d'Epstein-Barr [VEB], le virus de l'immunodéficience humaine [VIH], l'herpès, l'hépatite et la varicelle-zoster ;
(e) une infection parasitaire ;
(f) une infection parasitaire choisie dans l'ensemble constitué par la malaria et la tuberculose ; et
(g) le cancer.

5. Utilisation de IL-11 pour la fabrication d'un médicament destiné à induire une augmentation de l'activité des cellules T cytotoxiques chez un patient, comprenant le fait d'administrer IL-11 à une population *ex vivo* de cellules T cytotoxiques dudit patient, ce dernier souffrant d'au moins une des affections suivantes :
(a) un trouble viral aigu ;
(b) la grippe ;
(c) un trouble viral chronique ;
(d) un trouble viral chronique choisi dans l'ensemble constitué par le virus d'Epstein-Barr [VEB], le virus de l'immunodéficience humaine [VIH], l'herpès, l'hépatite et la varicelle-zoster ;
(e) une infection parasitaire ;
(f) une infection parasitaire choisie dans l'ensemble constitué par la malaria et la tuberculose ; et
(g) le cancer.

6. Utilisation d'une molécule d'ADN codant une protéine IL-11 pour la fabrication d'un médicament destiné à induire la production de cellules T cytotoxiques chez un patient, dans laquelle utilisation on transfecte une cellule T cytotoxique à l'aide de ladite molécule d'ADN et l'on cultive ladite cellule *ex vivo* dans des conditions dans lesquelles la protéine IL-11 est produite, ledit patient souffrant d'au moins une des affections suivantes :
(a) un trouble viral aigu ;
(b) la grippe ;
(c) un trouble viral chronique ;
(d) un trouble viral chronique choisi dans l'ensemble constitué par le virus d'Epstein-Barr [VEB], le virus de l'immunodéficience humaine [VIH], l'herpès, l'hépatite et la varicelle-zoster ; et
(e) une infection parasitaire ;
(f) une infection parasitaire choisie dans l'ensemble constitué par la malaria et la tuberculose ; et
(g) le cancer.
